# EUROPEAN PATENT SPECIFICATION

(11) **EP 2 432 424 B1**
(45) Date of publication and mention of the grant of the patent: **22.04.2020**
(21) Application number: 10718647.0
(22) Date of filing: 29.04.2010
(51) Int. Cl.: A61F 2/06, A61F 2/82

(54) **A VASCULAR GRAFT**
GEFÄSSERSATZ
GREFFON VASCULAIRE

(30) Priority: 19.05.2009 US 468169
(43) Date of publication of application: 28.03.2012
(73) Proprietor: Vascular Flow Technologies Limited, Dundee DD2 1TY (GB)
(72) Inventor: HOUSTON, John, Graeme, Tayside PH2 7AW (GB); DICK, John, Bruce, Cameron, Blairgowrie PH13 9AU (GB); HOOD, Robert, Gordon, Tayside DD2 5EF (GB); STONEBRIDGE, Peter, Arno, Tayside PH2 7AG (GB)
(74) Representative: Ouzman, Beverley Nicola Claire
(86) International application number: PCT/GB2010/000866
(87) International publication number: WO 2010/133821

(56) References cited:
- EP-A1- 1 759 667
- WO-A1-01/89419
- WO-A1-2005/092240
- WO-A2-2004/047908
- AT-B- 134 543

## Description

### Field of the Invention

The present invention relates to a tubular graft comprising an elongate conduit.

### Background of the Invention

It is known in the art to provide vascular grafts as artificial vascular prostheses to be implanted in individuals with diseased blood vessels. For example, if an individual is suffering from atherosclerosis then a section of blood vessel may be replaced with a vascular graft.

The problem with such vascular grafts is that they have a tendency to cause turbulence in the flow of the blood that they carry, particularly at the join between the vascular graft and the blood vessel at either end. This can result in plaque formation, reduced flow capacity and thromboses in the blood vessel.

WO-A-00/38591 discloses a vascular graft in which a tubular graft is provided with four equally spaced ridges or fins on the interior of the graft. Each ridge is in the form of an axially extending helix. The ridges induce helical flow to the blood passing through the vascular graft. The provision of helical blood flow reduces the turbulence of the blood in the vascular graft which, in turn, reduces the likelihood of plaque formation, reduced flow capacity and thromboses.

EP-A-1759667 discloses a tubular graft which comprises an internal helical formation or fin which imparts helical flow on blood passing through the graft. The internal helical formation terminates at the first and second ends of the tubular graft. At least one end of the tubular graft is tapered from an inner base to an outer tip, which assists a surgeon in suturing the tubular graft to a healthy section of blood vessel, during implantation of the graft.

WO-A-2005/092240 reports on a tubular graft which comprises an axially extending internal helical protrusion or fin, which imparts helical flow on blood passing through the graft, as well as an axially extending external helical formation which supports the graft. The internal helical protrusion has a different helix angle from the external helical formation. The tubular graft comprises a tubular portion made from expanded polytetrafluoroethylene (ePTFE) and thus the tubular portion is generally flexible. The internal helical fin is then formed by fitting the tubular portion onto a mandrel and within a mould and injecting molten polyurethane into the mould around the exterior of the tubular portion. A helical channel is present in the exterior of the mandrel such that the polyurethane deforms the tubular conduit by pressing it into the helical channel in the mandrel to create a helical fin on the inside surface of the tubular portion. Once the polyurethane cools and solidifies, the deformation of the tubular portion is held permanently in place and the finished graft comprises an ePTFE tubular portion with a polyurethane helical structure which deforms the ePTFE conduit to form an internal, axially extending helical fin. The external helical formation is formed in a similar manner but without deformation of the tubular portion since only an exterior structure is formed.

It has now been recognised that one problem with such vascular grafts is that, in practice, the helical polyurethane structure that forms the internal helical fin also has the effect of stiffening the vascular graft. In practice, it has been found this stiffening of the vascular graft makes it more difficult for a surgeon to manipulate the graft during the implantation procedure.

The present invention seeks to alleviate the above problem and arises from the separate realisation that helical flow can be imparted on blood passing through this type of vascular graft even when the internal helical fin does not extend along the entire length of the graft.

### Summary of the Invention

According to the present invention, there is provided a tubular graft comprising an elongate conduit having distal and proximal ends and a helical fin projecting inwardly from the inner surface of the elongate conduit and extending parallel to the axis of the elongate conduit, wherein the helical fin extends from the distal end of the elongate conduit to a point short of the proximal end of the elongate conduit.

Conveniently, the helical fin extends from the distal end of the elongate conduit for less than 50% of the entire length of the elongate conduit, preferably less than 25% thereof, more preferably less than 10% thereof.

Preferably, the helical fin forms between 50% and 150% of one complete rotation, preferably between 80% and 120%.

Advantageously, the helical fin has a helix angle of between 5° and 20° preferably a helix angle of 8°.

Conveniently, the helical fin extends parallel to the axis of the elongate conduit for a distance of between 8 and 12cm of the axis.

Preferably, the tubular graft further comprises an external helical formation located around the outside of the elongate conduit for supporting the elongate conduit.

Conveniently, the external helical formation has a helix angle greater than the helix angle of the helical fin.

Preferably, the external helical formation has a helix angle of between 65° and 80°.

Advantageously, the distal end is cut obliquely so that it has a tapered finish.

Conveniently, the proximal end is obliquely cut so that it has a tapered finish.

The helical fin imparts helical flow on blood passing through the elongate conduit.

In preferred embodiments, the elongate conduit is made from a flexible material (such as ePTFE) and the helical fin is formed from a generally rigid material, which may also be somewhat elastically deformable (such as polyurethane). The generally rigid material may be external of the elongate conduit and deform the elongate conduit such that the helical fin projects inwardly from the inner surface of the elongate conduit.

The terms "helix" and "helical" are used herein to cover the mathematical definitions of helix and helical and any combination of mathematical definitions of helical and spiral.

A "helix angle" referred to herein is the angle between the helix and the axial line about which it is formed, in particular the axis of the tubular graft.

### Brief Description of the Figure

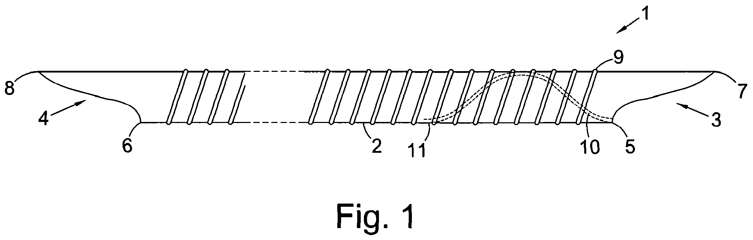

Figure 1 is partly-schematic side view of tubular graft in accordance with embodiment of the present invention.

### Detailed Description

Referring to Figure 1, a tubular vascular graft 1 comprises an elongate conduit 2 made from ePTFE having distal and proximal ends 3, 4. Each of the distal and proximal ends 3, 4 is cut obliquely at an angle of between 15° and 65° from the perpendicular. More specifically, each of the distal and proximal ends 3, 4 is tapered from an inner base 5, 6 to an outer tip 7, 8 such that the outer tip 7, 8 extends axially outwardly from the elongate conduit 2 further than the base 5, 6 and thus each outer tip 7, 8 forms a flap which overhangs its respective base 5, 6. Furthermore, when viewed from the side, as shown in Figure 1, the taper from each base 5, 6 to its respective tip 7, 8 is approximately sinusoidal, such that when the tubular graft is viewed from below, the orifice that forms the distal and proximal end 3, 4, is "egg-shaped". That is to say each orifice is approximately elliptic but has a curved end at its base 5, 6 with a relatively large radius of curvature (i.e. a blunt end) and a curved end at its tip 7, 8 with a relatively small radius of curvature (i.e. a sharp end). Further details of the configuration of the distal and proximal ends 3, 4 are provided in EP-A-1759667 which is hereby incorporated by reference.

Around the exterior of the elongate conduit 2 is a polyurethane support structure 9 which is in the form of an axially extending helix having a helix angle of greater than 50° and preferably between 65° and 80°. The support structure 9 extends from adjacent the base 5 of the distal end 3 to adjacent to the base 6 of the proximal end 4.

The elongate conduit 2 also comprises an internal helical fin 10, which extends axially from the base 5 of the distal end 3 and passes through one revolution before terminating at termination point 11 which is some distance from the base 6 of the proximal end 4. The internal helical fin 10 has a helix angle of between 5° and 20°, preferably of 8°. The internal helical fin 10 is formed by an external polyurethane structure (not shown) which deforms the elongate conduit as described in WO-A-2005/092240.

In this embodiment, the internal helical fin 10 is of bell-shaped cross section. However, this cross sectional shape of the internal helical fin 10 is not essential to the invention and in other embodiments, the fin is of a different cross sectional shape such as a "U"-shape as is disclosed in WO-A-03/045279 or a triangular shape as disclosed in WO-A-2005/004751.

The tubular graft 1 is used in an anastomosis procedure to replace a damaged section of blood vessel as will now be described. In order to implant the tubular graft 1, a healthy section of blood vessel is selected adjacent to the damaged section of blood vessel. An aperture is formed in the healthy section of blood vessel. The aperture is "egg-shaped" being approximately elliptical having a base which is curved with a relatively large radius of curvature and a tip which is curved with a relatively small radius of curvature. Thus the aperture is shaped to correspond to the shape of the orifice that forms the distal end 3 of the tubular graft 1 except that the aperture is slightly smaller than the orifice which forms the distal end 3.

In the next step of the procedure, the distal end 3 of the tubular graft 1 is located over the aperture of the healthy section of blood vessel. The surgeon then sutures the tubular graft 1 to the healthy section of blood vessel, joining the edge of the distal end 3 of the elongate conduit to the edge of the aperture. The egg-shape of the distal end 3 of the tubular graft 1 provides the surgeon with the maximum amount of material to carry out the suturing step, which assists the surgeon performing the procedure. Because of the distal end 3 of the elongate conduit 2 is cut obliquely, the tubular graft 1, at the distal end 3, meets the blood vessel at an angle of between 25° and 75°, depending on the angle of the oblique cut.

The step of forming an aperture is repeated in a second section of healthy blood vessel at the proximal end 4 of the damaged section of blood vessel. Similarly, the step of suturing the proximal end 4 of the tubular graft 1 to the second section of healthy blood vessel over the second aperture is performed just as for the distal end 3. The proximal end 4 of the tubular graft 1 also meets the blood vessel at an angle of between 25° and 75°, depending on the angle at which the proximal end 4 is cut obliquely.

While the procedure is taking place, blood is prevented from passing through the blood vessel being operated on but once the suturing of the tubular graft 1 to the blood vessel is completed, blood is allowed to pass through the blood vessel and into the tubular graft 1. The blood enters the tubular graft 1 at the proximal end 4. Helical flow is conferred on the blood by the internal helical fin 10 and subsequently the blood leaves the tubular graft 1 at the distal end 3. Because the internal helical fin 10 extends from the base 5 of the distal end 3 of the tubular graft 1, the internal helical fin 10 provides improved blood flow from the tubular graft 1 into the healthy section of blood vessel. This occurs because the internal helical fin 10 imparts spiral flow on the blood flowing through the tubular graft 1 and this reduces turbulence in the junction between the tubular graft 1 and the healthy section blood vessel, minimising cell damage and plaque build up. The damaged section of blood vessel is usually occluded and totally incorporated into the surrounding tissue, but occasionally it is removed.

It is to be appreciated that, while the support structure 9 prevents kinking of the elongate conduit 2, because the internal helical fin 10 extends for only a relatively short length of the elongate conduit 2, stiffening of the elongate conduit 2 is minimised. This makes the surgical implantation of the tubular graft easier for the surgeon since the tubular graft 1 can be more easily manipulated into position at the distal and proximal ends 3, 4. Furthermore, one revolution of the internal helical fin 10 is sufficient to impart helical flow on blood passing through the vascular graft 1 as shown in the Example herein. It has been found that the effect of helical flow imparted on blood is most usefully imparted at the distal end 3 of the tubular graft 1 because, without the presence of the internal helical fin 10, there tends to be a build up of tissue at the distal end 3 which can ultimately occlude the tubular graft 1.

In the above-described embodiment, the internal helical fin 10 comprises a single revolution (that is to say it turns a full 360° along its length). However, in alternative embodiments, the internal helical fin 10 may be somewhat shorter or longer than this and may, for example, be in the range of 50% to 150% or 80% to 120% of a single revolution. What is important is that the termination point 11 of the internal helical fin 10 is well short of the base 6 of the proximal end 4 of the vascular graft 1 such that the majority, or at least a significant portion, of the elongate conduit 2 does not have the internal helical fin 10 extending through it. Depending upon the helix angle of the internal helical fin 10 and the dimensions of the vascular graft 1, the length of the elongate conduit 2 over which the internal helical fin 10 extends varies but is typically in the range of between 8 and 12 cm. For example, the helical fin 10 may extend from the distal end 3 of the elongate conduit 2 for less than 50%, 25% or 10% of the entire length of the elongate conduit 2 (the "entire length" being the distance between the respective bases 5, 6 of the distal and proximal ends 3, 4).

It is to be understood that, although it is preferred, is not essential to the invention that the internal helical fin 10 extends precisely from the base 5 of the distal end 3. In some alternative embodiments, the internal helical fin 10 extends from within 90° either side of the base 5 of the distal end 3.

### EXAMPLE

### Introduction

Short flow tests of a 100 mm long straight graft were carried out and confirm that only a single revolution of an internal helical fin in a graft is required in order to impart helical flow on liquid flowing through the graft.

### Aim

To take ultrasound measurements upstream and downstream of a test graft to determine the swirl number (peak transverse velocity versus linear velocity maximum) and the presence of C.T.F.S. (Characteristics Transverse Flow Signature) downstream from a 100 mm long straight graft with and without spiral inserts upstream from the grafts.

### Objective

To compare this 100 mm straight graft with 100 mm spiral grafts and with C.F.D. (Computational Fluid Dynamics) results.

### Equipment

Networked computer equipment (TCT computer and accessories)
Ultrasound equipment (GE LOGIQ 400 CL with Sony camera and accessories)
Flow pump and water bath equipment (Braveheart)

| | |
|---|---|
| Grafts: | 1 x 100mm length, no internal fin |
| | 1 x 100mm length with an internal helical fin having an 8° helix angle |
| | and forming approx. 83% of a complete revolution |
| | 1 x 100mm length with an internal helical fin having a 17 ° helix angle |
| | and forming approx. 83% of a complete revolution. |

### Spiral Inserts

### 1. Spiral Insert A

Description: no fins, 100mm effective length, latex coated fabric material, 8mm diameter.

### 2. Spiral Insert B

Description: 21 deg fin angle, 2.5mm fin depth, p3 profile, 1, fin, 60mm effective length (linear fin length), aluminium material, 8mm diameter, followed by, no fins, 100mm effective length, latex coated fabric material, 8mm diameter.

### 3. Spiral Insert C

Description: -21 deg fin angle, 2.5mm fin depth, p3 profile, 1 fin, 60mm effective length (linear fin length), aluminium material, 8mm diameter, followed by, no fins, 100mm effective length, latex coated fabric material, 8mm diameter.

| | |
|---|---|
| Environmental Conditions: | Standard room temperature and pressure. |
| Pump Set Up: | CONSTANT 17 mL/s: constant flow of 17 mL/s. |

References: Kang and Bonneau 2003, and Cooney 1976 in Biomedical Engineering Principles.

The swirl numbers (peak transverse velocity versus linear velocity maximum) are given in Table 1.

**Table 1**

| | |
|---|---|
| Spiral Insert C | -0.002 |
| Spiral Insert A | 0.011 +/1-0.009 |
| Spiral Insert B | 0.039 +/-0.016 |

Observations of the presence or absence of C.T.F.S. (Characteristic Transverse Flow Signature) are given in Table 2.

**Table 2**

| | Control graft | 8 deg graft | 17 deg graft |
|---|---|---|---|
| -21 deg insert | No C.T.F.S. | No. C.T.F.S. | No. C.T.F.S. |
| No insert | No. C.T.F.S. | C.T.F.S. | C.T.F.S. |
| 21 deg insert | Undetermined | Undetermined | C.T.F.S. |

### Conclusion

C.T.F.S. (Characteristic Transverse Flow Signature), that is to say, coherent flow was observed downstream from the spiral grafts with and without the 21 deg insert. This indicates that a single revolution of the internal helix fin of a graft is capable of conferring helical flow of fluid passing through the graft.

## Claims

1. A tubular graft (1) comprising an elongate conduit (2) having first and second ends (4, 3) and a helical fin (10) projecting inwardly from the inner surface of the elongate conduit (2), where the axis of said helical fin (10) extends parallel to the axis of the elongate conduit (2), and wherein the helical fin (10) has a helix angle of between 5° and 20°, **characterised in that** the helical fin (10) extends from the second end (3) of the elongate conduit (2) for less than 50% of the entire length of the elongate conduit (2).

2. A tubular graft (1) according to claim 1 wherein the helical fin (10) extends from the second end (3) of the elongate conduit (2) for less than 25% of the entire length of the elongate conduit (2), preferably less than 10% thereof.

3. A tubular graft (1) according to claim 1 or 2 wherein the helical fin (10) forms between 50% and 150% of one complete rotation, preferably between 80% and 120%.

4. A tubular graft (1) according to any one of the preceding claims wherein the axis of the helical fin (10) extends parallel to the axis of the elongate conduit (2) for a distance of between 8 and 12cm of the axis of the elongate conduit (2).

5. A tubular graft (1) according to any one of the preceding claims further comprising an external helical formation (9) located around the outside of the elongate conduit (2) for supporting the elongate conduit (2).

6. A tubular graft (1) according to claim 5 wherein the external helical formation (9) has a helix angle greater than the helix angle of the helical fin (10).

7. A tubular graft (1) according to claim 5 or 6 wherein the external helical formation (9) has a helix angle of between 65° and 80°.

8. A tubular graft (1) according to any one of the preceding claims wherein the second end (3) is cut obliquely so that it has a tapered finish.

9. A tubular graft (1) according to any one of the preceding claims wherein the first end (4) is cut obliquely so that it has a tapered finish.

## Patentansprüche

1. Schlauchförmiger Ersatz (1), umfassend eine längliche Leitung (2) mit ersten und zweiten Enden (4, 3) und einer von der Innenfläche der länglichen Leitung (2) nach innen vorstehenden schraubenförmigen Rippe (10), wobei sich die Achse der schraubenförmigen Rippe (10) parallel zur Achse der länglichen Leitung (2) erstreckt, und wobei die schraubenförmige Rippe (10) einen Schraubenwinkel von zwischen 5° und 20° aufweist, **dadurch gekennzeichnet, dass** sich die schraubenförmige Rippe (10) über weniger als 50 % der Gesamtlänge der länglichen Leitung (2) vom zweiten Ende (3) der länglichen Leitung (2) erstreckt.

2. Schlauchförmiger Ersatz (1) nach Anspruch 1, wobei sich die schraubenförmige Rippe (10) über weniger als 25 % der Gesamtlänge der länglichen Leitung (2), vorzugsweise weniger als 10 % davon, vom zweiten Ende (3) der länglichen Leitung (2) erstreckt.

3. Schlauchförmiger Ersatz (1) nach Anspruch 1 oder 2, wobei die schraubenförmige Rippe (10) zwischen 50 % und 150 % der vollständigen Rotation, vorzugsweise 80 % und 120 % formt.

4. Schlauchförmiger Ersatz (1) nach einem der vorangehenden Ansprüche, wobei sich die Achse der schlauchförmigen Rippe (10) über einen Abstand von zwischen 8 und 12 cm der Achse der länglichen Leitung (2) parallel zur Achse der länglichen Leitung (2) erstreckt.

5. Schlauchförmiger Ersatz (1) nach einem der vorangehenden Ansprüche, ferner umfassend eine externe schraubenförmige Formierung (9), die sich um die Außenseite der länglichen Leitung (2) befindet, um die längliche Leitung (2) zu unterstützen.

6. Schlauchförmiger Ersatz (1) nach Anspruch 5, wobei die externe schraubenförmige Formierung (9) einen Schraubenwinkel aufweist, der größer als der Schraubenwinkel der schraubenförmigen Rippe (10) ist.

7. Schlauchförmiger Ersatz (1) nach Anspruch 5 oder 6, wobei die externe schraubenförmige Formierung (9) einen Schraubenwinkel von zwischen 65° und 80° aufweist.

8. Schlauchförmiger Ersatz (1) nach einem der vorangehenden Ansprüche, wobei das zweite Ende (3) schräg geschnitten ist, so dass es eine konische Oberfläche aufweist.

9. Schlauchförmiger Ersatz (1) nach einem der vorangehenden Ansprüche, wobei das zweite Ende (4) schräg geschnitten ist, so dass es eine konische Oberfläche aufweist.

## Revendications

1. Greffon tubulaire (1) comprenant un conduit allongé (2) possédant des première et seconde extrémités (4, 3) et une ailette hélicoïdale (10) faisant saillie vers l'intérieur à partir de la surface interne du conduit allongé (2), où ledit axe de ladite ailette hélicoïdale (10) s'étend parallèle à l'axe du conduit allongé (2), et ladite ailette hélicoïdale (10) possédant un angle d'hélice compris entre 5° et 20°, **caractérisé en ce que** l'ailette hélicoïdale (10) s'étend à partir de la seconde extrémité (3) du conduit allongé (2) sur moins de 50 % de toute la longueur du conduit allongé (2).

2. Greffon tubulaire (1) selon la revendication 1, ladite ailette hélicoïdale (10) s'étendant à partir de la seconde extrémité (3) du conduit allongé (2) sur moins de 25 % de toute la longueur du conduit allongé (2), de préférence moins de 10 % de celle-ci.

3. Greffon tubulaire (1) selon la revendication 1 ou 2, ladite ailette hélicoïdale (10) formant entre 50 % et 150 % d'une rotation complète, de préférence entre 80 % et 120 %.

4. Greffon tubulaire (1) selon l'une quelconque des revendications précédentes, ledit axe de l'ailette hélicoïdale (10) s'étendant parallèlement à l'axe du conduit allongé (2) sur une distance comprise entre 8 et 12 cm de l'axe du conduit allongé (2).

5. Greffon tubulaire (1) selon l'une quelconque des revendications précédentes, comprenant en outre une formation hélicoïdale externe (9) située autour de l'extérieur du conduit allongé (2) pour supporter le conduit allongé (2).

6. Greffon tubulaire (1) selon la revendication 5, ladite formation hélicoïdale externe (9) possédant un angle d'hélice supérieur à l'angle d'hélice de l'ailette hélicoïdale (10).

7. Greffon tubulaire (1) selon la revendication 5 ou 6, ladite formation hélicoïdale externe (9) possédant un angle d'hélice compris entre 65° et 80°.

8. Greffon tubulaire (1) selon l'une quelconque des revendications précédentes, ladite seconde extrémité (3) étant coupée obliquement afin qu'elle présente une finition effilée.

9. Greffon tubulaire (1) selon l'une quelconque des revendications précédentes, ladite première extrémité (4) étant coupée obliquement de sorte qu'elle présente une finition effilée.
